# EUROPÄISCHE PATENTSCHRIFT

(11) **EP 0 914 307 B1**
(45) Veröffentlichungstag und Bekanntmachung des Hinweises auf die Patenterteilung: **15.09.2004**
(21) Anmeldenummer: 97930430.0
(22) Anmeldetag: 27.06.1997
(51) Int. Cl.: C07B 61/00, C12P 1/00, C12P 13/04

(54) **ENZYMATISCH SPALTBARE LINKER FÜR FESTPHASENSYNTHESEN**
ENZYMATICALLY REMOVABLE LINKERS FOR SOLID PHASE SYNTHESIS
SEGMENTS DE LIAISON SEPARABLES PAR VOIE ENZYMATIQUE POUR SYNTHESES EN PHASE SOLIDE

(30) Priorität: 03.07.1996 DE 19626762
(43) Veröffentlichungstag der Anmeldung: 12.05.1999
(73) Patentinhaber: BASF AKTIENGESELLSCHAFT, 67056 Ludwigshafen (DE)
(72) Erfinder: WALDMANN, Herbert, D-76764 Rheinzabern (DE); SAUERBREI, Bernd, D-76137 Karlsruhe (DE); GRETHER, Uwe, D-76229 Karlsruhe (DE)
(86) Internationale Anmeldenummer: PCT/EP1997/003379
(87) Internationale Veröffentlichungsnummer: WO 1998/001406

(56) Entgegenhaltungen:
- WO-A-97/20855
- H. WALDMANN ET AL.: "Synthesis of the palmitoylated snd farnesylated C-terminal lipohexapeptide of the human N-Ras protein by employing an enzymatically removable urethane protecting group" ANGEWANDTE CHEMIE, INTERNATIONAL EDITION, Bd. 34, Nr. 20, 1995, WEINHEIM, DE, Seiten 2259-62, XP002042719 in der Anmeldung erwähnt
- D.T. ELMORE ET AL.: "An enzyme-scissible linker for solid-phase peptide synthesis" JOURNAL OF THE CHEMICAL SOCIETY, CHEMICAL COMMUNICATIONS, Nr. 14, 1992, LONDON, GB, Seiten 1033-4, XP000653704 in der Anmeldung erwähnt

## Beschreibung

Die Erfindung betrifft enzymatisch spaltbare Linker für Festphasensynthesen, ein Verfahren zu ihrer Herstellung und ihre Verwendung.

Für die moderne Wirkstoffsuchforschung werden eine Vielzahl von molekularen Testsystemen wie beispielsweise Rezeptorbindungsassays, Enzymassays und Zell-Zellinteraktionsassays entwickelt. Die Automatisierung und Miniaturisierung dieser Testsysteme ermöglicht die Testung einer immer größeren Anzahl von Chemikalien auf ihre biologische Wirkung im Zufalls-Screening und damit auf eine mögliche Verwendung als Leitstruktur für einen Wirkstoff in der Medizin, Tiermedizin oder im Pflanzenschutz.

Die klassische Synthesechemie wurde durch diese Entwicklung zum limitierenden Faktor in der Wirkstoffsuchforschung.

Soll die Leistungsfähigkeit der entwickelten Testsysteme voll ausgeschöpft werden, muß die Effizienz der chemischen Wirkstoffsynthese beträchtlich gesteigert werden.

Zu dieser erforderlichen Effizienzsteigerung kann die kombinatorische Chemie einen Beitrag leisten, insbesondere wenn sie sich automatisierter Festphasensynthesemethoden bedient (s. z.B. Übersichtsartikel J. Med. Chem. 1994, 37, 1233 und 1994, 37, 1385).

Das Prinzip dieser kombinatorischen Synthesen beruht darauf, daß auf jeder Synthesestufe nicht nur mit einem Synthesebaustein, sondern mit mehreren, entweder parallel oder in Mischung umgesetzt wird. Auf jeder Stufe bilden sich alle möglichen Kombinationen, so daß mit relativ wenigen Bausteinen schon nach einigen Stufen eine große Anzahl von Produkten, eine sogenannte Substanzbibliothek entsteht.

Die Synthese an der Festphase hat den Vorteil, daß Nebenprodukte und überschüssige Reaktanden leicht entfernt werden können, so daß keine aufwendige Reinigung der Produkte notwendig ist. Durch hohe Überschüsse des gelösten Reaktionspartners lassen sich Reaktionen beschleunigen und die Umsetzungen optimieren. Die fertigen Syntheseprodukte können direkt, d.h. trägergebunden, oder nach Abspaltung von der festen Phase dem Massenscreening zugeführt werden. Auch Zwischenprodukte können im Massenscreening geprüft werden.

Bisher beschriebene Anwendungen beschränken sich überwiegend auf das Peptid- und Nukleotidgebiet (Lebl et al., Int. J. Pept. Prot. Res. 41, 1993: 203 und WO 92/00091) oder ihre Derivate (WO 96/00391). Da Peptide und Nukleotide als Pharmaka wegen ihrer ungünstigen pharmakologischen Eigenschaften nur begrenzt einsetzbar sind, ist es wünschenswert, die von der Peptid- und Nukleotidchemie her bekannten und bewährten Festphasensynthesemethoden für die synthetische organische Chemie zu nutzen.

In US 5 288 514 wird über eine der ersten kombinatorischen Festphasensynthesen in der organischen Chemie außerhalb der Peptidund Nukleotidchemie berichtet. US 5 288 514 beschreibt die sequentielle Synthese von 1,4-Benzodiazepinen an fester Phase.

WO 95/16712, WO 95/30642 und WO 96/00148 beschreiben weitere Festphasensynthesen potentieller Wirkstoffe in der kombinatorischen Chemie.

Um die Möglichkeiten der modernen Testsysteme im Massenscreening voll ausnutzen zu können, ist es jedoch notwendig, ständig neue Verbindungen mit einer möglichst hohen strukturellen Diversität in das Massenscreening einzuspeisen. Durch dieses Vorgehen läßt sich die Zeit zur Identifizierung und Optimierung einer neuen Wirkstoffleitstruktur beträchtlich verkürzen.

Es ist deshalb erforderlich, ständig neue diverse, kombinatorische Festphasensynthesen zu entwickeln.

Für diese neuen Synthesen ist es wichtig, daß die einzelnen Bausteine der Festphasenysnthese optimal aufeinander abgestimmt sind. Durch die Wahl der festen Phase wie beispielsweise Glas, Keramik oder Harze sowie des Linkers wird die Folgechemie am Träger entscheidend beeinflußt.

Um ein möglichst großes Spektrum organischer Synthesen an fester Phase betreiben zu können, besteht ein beträchtlicher Entwicklungsbedarf nach neuen festen Phasen sowie neuen Linker- bzw. Ankergruppen.

Bislang werden basen- oder säurelabile Linkergruppen verwendet, deren Abspaltungsbedingungen für viele am Träger synthetisierten Substanzen zu drastisch sind. Es werden deshalb große Anstrengungen unternommen, um Linker zu konstruieren, die unter milderen Bedingungen von der festen Phase abgespalten werden können.

Dabei wäre es wünschenswert, Enzyme für die Spaltung der Linker, unter milden Bedingungen verwenden zu können, wie dies schon für Schutzgruppen in einzelnen Fällen möglich ist. Ein Beispiel für eine enzymatisch spaltbare Schutzgruppe beschreibt Waldmann et al. in Angew. Chem. 1995, 107, Nr. 20: 2425-2428.

Von Elmore et al. wird ein erster enzymatisch spaltbarer Linker für die Peptidsynthese an fester Phase (J. Chem. Soc., Chem. Commun., Vol. 14, 1992: 1033-1034), der unter milden Bedingungen vom Träger gespalten werden kann, beschrieben. Von Schuster et al. wird ein weiterer enzymatisch spaltbarer Linker für Festphasensynthesen von Zuckern beschrieben (J. Am. Chem. Soc., 1994, 116: 1135-1136 und US 5369017).

Nachteil beider Methoden ist, daß nach der enzymatischen Spaltung im Produkt stets Teile des Linkers verbleiben. Beide Methoden sind außerdem bezüglich der Linker spaltenden Enzyme stark beschränkt, so verwendet Elmore zur Spaltung Kalbsmilz-Phosphodiesterase und Schuster et al. beschreibt Serinproteasen zur Spaltung.

Aufgabe der vorliegenden Erfindung war es, einen unter milden Bedingungen spaltbaren Linker zu entwickeln, der die oben genannten Nachteile nicht aufweist und eine breite Palette von organischen Synthesen an fester Phase ermöglicht.

Die gestellte Aufgabe wurde durch einen enzymatisch spaltbaren Linker, gebunden an eine feste Phase, an dem über eine funktionelle Gruppe organische Verbindungen synthetisiert werden, dadurch gekennzeichnet, daß der Linker eine Erkennungsstelle für ein hydrolytisches Enzym enthält und durch Reaktion mit dem Enzym so zerfällt, daß keine Molekülteile des Linkers im synthetisierten Produkt verbleiben und daß die Erkennungsstelle für das Enzym und die Stelle, an der das Syntheseprodukt über Zerfall des Linkers freigesetzt wird, verschieden sind, gelöst.

Die Erfindung betrifft außerdem die Herstellung der Linker sowie ihre Verwendung.

Ein bevorzugter Linker wird durch die allgemeine Formel I 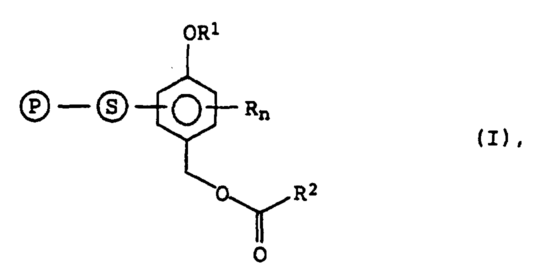 in der die Variablen und Substituenten folgende Bedeutung haben:
- (P): eine feste Phase
- (S): ein Spacer, mit einer Länge, die 1 bis 30 Methylengruppen entspricht
- R: Wasserstoff oder ein unter den Reaktionsbedingungen inerter Rest
oder zwei benachbarte inerte Reste R, die zusammen einen aromatischen, heteroaromatischen oder aliphatischen Ring bilden
- R¹: substituiertes oder unsubstituiertes C₁-C₂₀-Alkyl-, C₃-C₂₀-Alkenyl-, C₃-C₆-Alkinyl-, C₁-C₂₀-Alkylcarbonyl-, C₁-C₂₀-Alkylphosphoryl-, C₃-C₂₀-Alkenylcarbonyl-, C₃-C₆-Alkinylcarbonyl-, C₃-C₂₀-Alkenylphosphoryl-, C₃-C₆-Alkinylphosphoryl-, C₃-C₂₀-Cycloalkyl-, C₃-C₂₀-Cycloalkylcarbonyl-, C₃-C₂₀-Cycloalkylphosphoryl-, Aryl-, Arylcarbonyl-, Arylphosphoryl-, Hetaryl-, Hetarylcarbonyl-, Hetarylphosphoryl-, Glycosyl-, substituierte oder unsubstituierte Aminosäuren oder Peptide
- R²: eine nucleofuge Gruppe
- n: 1 oder 2, charakterisiert.

Erfindungsgemäße Linker sind Linker, die eine Erkennungsstelle für ein hydrolytisches Enzym enthalten und durch Reaktion mit dem Enzym so zerfallen, daß der Linker vollständig aus einem an der Festphase über den Linker gebundenen synthetisierten Produkt abgespalten wird, d.h. es verbleiben keine Molekülteile des Linkers im synthetisierten Produkt.

Bevorzugt wird der Linker unter CO₂-Abspaltung vom an der Festphase synthetisierten Produkt abgespalten.

Unter Erkennungsstelle für ein Enzym ist eine Bindung zu verstehen, die durch ein hydrolytisches Enzym spaltbar ist. Durch hydrolytische Enzyme spaltbare Bindungen sind beispielsweise Bindungen wie Ester-, Säureamid-, Ether-, Phosphorsäureesteroder Glycosidbindungen.

Als Enzyme zur Spaltung des erfindungsgemäßen Linkers unter milden Bedingungen sind hydrolytische Enzyme wie Lipasen, Esterasen, Amidasen, Proteasen, Peptidasen, Phosphatasen, Phospholipasen, Peroxidasen oder Glycosidasen geeignet. Bevorzugt sind Enzyme ausgewählt aus der Gruppe Lipasen, Esterasen, Amidasen, Proteasen oder Glycosidasen, besonders bevorzugt Lipasen, Esterasen oder Glycosidasen.

Beispielhaft werden erfindungsgemäße Linker in Formel IV (Schema A) dargestellt. 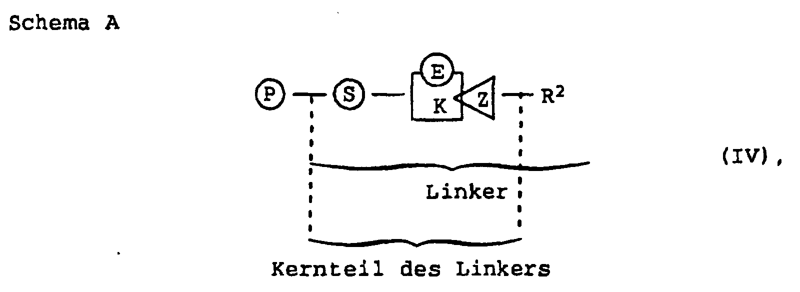 wobei die Substituenten und Variablen folgende Bedeutung haben:
- (P): eine feste Phase
- (S): ein Spacer mit einer Länge, die 1 bis 30 Methylengruppen entspricht
- (E): Erkennungsstelle für ein hydrolytisches Enzym
- (K): Zentrale Linkerstruktur
- (Z): funktionelle Gruppe, an der das Produkt freigesetzt wird
- R²: nucleofuge Gruppe, über die die Synthese der Produkte am Linker erfolgt.

Über die zentrale Linkerstruktur sind die Enzymerkennungsstelle (E), die feste Phase (P) über den Spacer (S) und die Stelle (Z), an der das Produkt freigesetzt wird, verbunden.

Für den Aufbau des Linkers müssen mindestens drei Funktionalitäten im Molekül enthalten oder in das zentrale Molekül einführbar sein. Über die Funktionalitäten werden die Enzymerkennungsstelle, die feste Phase und die funktionelle Gruppe, an der das Produkt an den Linker gebunden wird, verbunden. Darüber hinaus bestehen keine Beschränkungen hinsichtlich der chemischen Struktur des zentralen Linkers.

Die zentrale Linkerstruktur kann aus ggf. substituierten aliphatischen, aromatischen oder heteroaromatischen Strukturen oder deren Kombinationen aufgebaut sein. Bevorzugt enthält die zentrale Linkerstruktur aromatische Strukturen, beispielsweise einen Phenyl- oder Naphthylring.

Der Linker enthält neben der festen Phase, dem Spacer, der Enzymerkennungsstelle und der funktionellen Gruppe, an der das Produkt freigesetzt wird (= Kernteil des Linkers), eine nucleofuge Gruppe (R²), über die die Anbindung der Syntheseprodukte erfolgt.

Der Linker ermöglicht eine milde und selektive Abspaltung der Syntheseprodukte vom Trägermaterial, ohne daß die Syntheseprodukte zerstört oder verändert werden.

Dabei sind pH-Bereiche von pH 2,0 bis 10,0 bevorzugt von pH 4,0 bis 8,0, sowie Temperaturbereiche von -10°C bis 100°C bevorzugt von 15°C bis 50°C für die enzymatische Abspaltung des Produkts vom Linker vorteilhaft. Die Abspaltung kann in wäßriger Lösung bis zu fast reinem Lösungsmittel mit Spuren an Wasser erfolgen. Bevorzugt erfolgt die Abspaltung mit einem Lösungsmittelanteil zwischen 10 und 50 Gew.-%.

Zum Aufbau des Linkers an fester Phase ist diese gegebenenfalls in dem Fachmann bekannter Weise zu modifizieren.

Der Linker ist, je nachdem welche feste Phase verwendet werden soll, über eine Ester-, Ether-, Amid-, Amin-, Sulfid- oder Phosphatbindung an die feste Phase gebunden.

Die Bindung an die feste Phase erfolgt dabei in an sich bekannter Weise.

So wird beispielsweise die Anbindung an Merrifield-Harz bzw. an 2-Chlortrityl-Harz von Verbindungen mit freien Hydroxylgruppen in P.M. Worster et al. (Angew. Chem. Int. Ed. Engl. 1979, 18, 221 ff) bzw. in C. Chen et al. (J. Am. Chem. Soc. 1994, 116, 2661-2662) beschrieben.

Die Anbindung über eine Aminobindung wird beispielsweise in M. Cardno et al. (J. Chem. Soc., Chem. Commun. 1995, 2163 ff) für 2-Chlortrityl-Harz, in E. Bayer (Angew. Chem. 1991, 103, 117 ff) für Nova Syn® TG Carboxy-Harz, in J. R. Hanske et al. (Tetrahedron Lett., 1995, 36, 1589-1592) für Wang- oder Tentagel® S PHB-Harz beschrieben.

Eine Anbindung an den Träger über Thiolgruppen wird beispielsweise für Merrifield-Harz in Reynolds et al. (US 5 324 483) beschrieben.

Die hier genannten Anbindungsbeispiele, die dem Fachmann wohl bekannt sind, sind hier nur als beispielhafte Reaktionen aufgeführt, weitere Anbindungsmöglichkeiten sind dem Fachmann bekannt (Lit. Calbiochem-Novabiochem - The Combinatorial-Chemistry Catalog Feb. 1996, 1-26 und S1-S24).

Im bevorzugten Linker der allgemeinen Formel I wird durch die Spaltung der Enzymerkennungsstelle durch beispielsweise Enzyme wie Lipasen, Esterasen, Amidasen, Proteasen oder Glycosidasen ein Zerfall des Linkers ausgelöst. Durch die enzymatische Spaltung des Linkers entsteht zunächst ein Phenolat, welches spontan in ein festphasengebundenes Chinonmethid und CO₂ weiter zerfällt. Dabei wird das Produkt, das keine Reste des Linkers enthält, freigesetzt.

Beim bevorzugten Linker der Formel I handelt es sich um ein phenyloges Acetal. Weitere erfindungsgemäße Linker können auch daraus abgeleitete vinyloge oder normale Acetale enthalten (s. Reaktion 2).

Vorteilhafte erfindungsgemäße Linker zerfallen nach enzymatischer Spaltung beispielsweise unter Bildung eines Lactams oder Lactons und setzen so das Produkt ohne Linkerreste frei. Die folgenden Reaktionen 1 und 2 sollen diese allgemeinen Zerfallsprinzipien beispielhaft veranschaulichen:

### 1. Reaktion

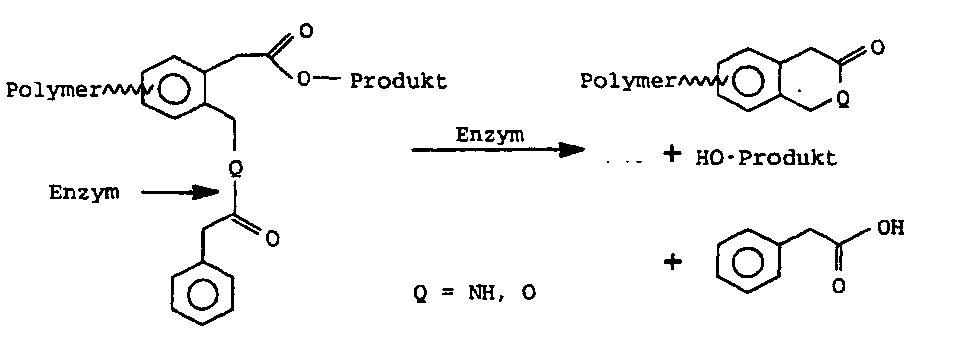

### 2. Reaktion

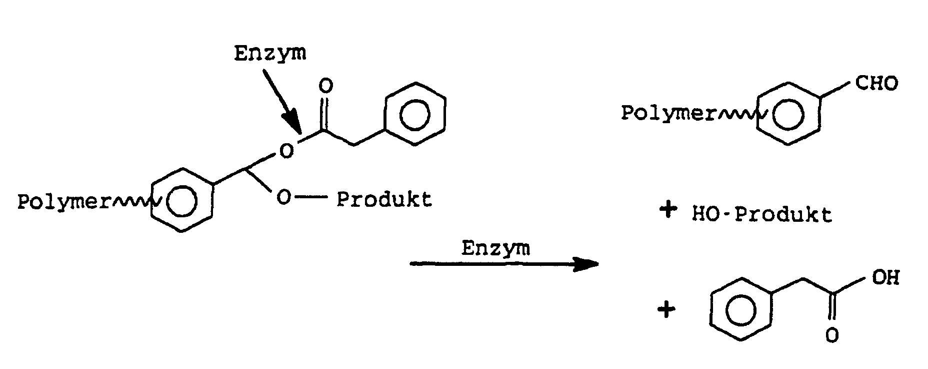

Diese in den Reaktionen 1 und 2 veranschaulichten Zerfallsprinzipien sind nicht auf die dort gezeigten Enzymerkennungsstellen wie z.B. Lipasen oder Amidasen beschränkt.

Erfindungsgemäße Linker zeichnen sich durch eine räumliche Distanz zwischen der Enzymerkennungsstelle und der Stelle, an der das Produkt über Zerfall des Linkers freigesetzt wird, aus, d.h. Enzymerkennungsstelle und die Stelle, an der das Produkt freigesetzt wird, sind unterschiedlich. Dadurch wird eine sterische Beeinträchtigung der enzymatischen Umsetzung durch das Substrat weitestgehend ausgeschlossen. Vorteilhafterweise liegt eine Distanz ausgedrückt in Methylengruppeneinheiten von 2 bis 8 Methyleneinheiten zwischen der Enzymerkennungsstelle und der Stelle, an der das Produkt freigesetzt wird, bevorzugt 4 bis 8 Methyleneinheiten.

Die erfindungsgemäßen Linker werden durch eine Vielzahl von Enzymen unter milden Bedingungen vollständig vom Produkt abgespalten und verbleiben an der festen Phase.

Als feste Phase (P) können für die erfindungsgemäßen Linker prinzipiell alle Träger, wie sie zum Beispiel aus der Festphasen-Peptidsynthese oder -Nucleinsäurensynthese bekannt sind, verwendet werden.

Nutzbare Träger können, soweit sie mit der verwendeten Synthesechemie in der Kombinatorik und der Anknüpfung des Linkers an die feste Phase kompatibel sind, aus einer Vielzahl von Materialien bestehen. Die Größe der Träger kann je nach Material in weitem Rahmen variiert werden. Bevorzugt werden Partikel im Bereich von 1 µm bis 1,5 cm als Träger verwendet, besonders bevorzugt werden bei polymeren Trägern Partikel im Bereich zwischen 1 µm und 150 µm. Aber auch Gele sind geeignet.

Die Form der Träger ist beliebig, bevorzugt sind sphärische Partikel. Die Träger können in ihrer Größenverteilung homogen oder heterogen sein, bevorzugt sind homogene Partikelgrößen.

Es können auch gegebenenfalls Mischungen verschiedener Partikel verwendet werden.

Trägermaterialien, die wenig oder gar nicht kompressibel sind, sind gegenüber kompressiblen Materialien bevorzugt, wenn beispielsweise ein Abtrennung des trägergebundenen Produkts z.B. über Zentrifugation oder eine Produktsynthese in Durchflußreaktoren durchgeführt werden soll, d.h. die verwendeten Träger sollten vorteilhafterweise eine gewisse Druckstabilität und ein günstiges Sedimentationsverhalten besitzen.

Von Vorteil ist bei längerer mechanischer Belastung auch eine günstige Abriebfestigkeit der verwendeten Träger.

Vorteilhafte Träger sollten poröse Materialen wie beispielsweise Sinterglas, Sintermetalle, poröse Keramiken oder Harze sein, die eine große innere Oberfläche in einem Bereich von 5 bis 2000 m²/g Trägermaterial, bevorzugt 40 bis 800 m²/g, besonders bevorzugt 50 bis 500 m²/g haben. Der Porendurchmesser der Materialen sollte vorteilhafterweise so gewählt werden, daß keine Stofftransportlimitierungen durch Diffusion oder durch aktiven Stofffluß entstehen. Der Porendurchmesser liegt zweckmäßigerweise zwischen 10 nm bis 500 nm, bevorzugt zwischen 30 nm bis 200 nm.

Die verwendeten Trägermaterialien sollten vorteilhafterweise ein möglichst großes Porenvolumen (> 1 ml/g Trägermaterial) besitzen.

Es können natürliche, anorganische oder organische Materialien verwendet werden.

Geeignete feste Phasen (P) sind beispielsweise funktionalisierte Partikel ausgewählt aus der Gruppe Keramik, Glas, Latex, quervernetzte Polystyrole, quervernetzte Polyacrylamide oder andere Harze, natürliche Polymere, Gold, kolloidale Metallpartikel, Silicagele, Aerogele oder Hydrogele.

Die Linker können auf der Oberfläche der festen Phase oder im Inneren der festen Phase oder auf beidem gebunden sein.

Unter Latizes sind kolloidale Dispersionen von Polymeren in wäßrigen Medien zu verstehen.

Es kann sich dabei um natürliche oder synthetische Latizes oder Mikrolatizes handeln, die beispielsweise durch Emulsionspolymerisation geeigneter Monomere oder durch Dispergieren von Polymeren in geeigneten Lösungsmitteln hergestellt wurden.

Unter quervernetzten Polystyrolen, quervernetzte Polyacrylamide oder anderen Harzen sind beispielsweise Polyacrylamid, Polymethacrylamid, Polyhydroxyethylmethacrylat, Polyamid, Polystyrol, (Meth)acrylat-Copolymere von beispielsweise (Meth)-acrylsäure, (Meth)acrylsäureestern und/oder Itaconsäure, Crotonsäure, Maleinsäure, PU-Schäume, Epoxydharze oder sonstige Copolymere zu verstehen.

Als natürliche Polymere bzw. Träger seien beispielsweise Agarose, Cellulose, Alginat, Chitosan, Dextran, Levan, Xanthan, Collagen, Gellan, X-Carrageenan, Agar, Pectin, Ramanian, Holzschnitzel, mikrokristalline Cellulose, Hexosamine oder Gelatine genannt.

Ebenfalls geeignete Träger sind Kieselerde, Kieselgur, Metalloxide oder Blähton.

Die Auswahl des geeigneten Trägers ist abhängig von der Chemie zur Verknüpfung des Linkers mit der festen Phase und von der nachher durchgeführten Synthesechemie. Gruppen die mit dieser Chemie nicht kompatibel sind, werden in dem Fachmann bekannter Weise geschützt.

Für die Auswahl des geeigneten Trägers spielt außerdem eine Rolle, daß der Träger vorteilhafterweise keine Gruppen oder Ionen oder sonstige Moleküle enthält, die die zur Abspaltung des Linkers verwendeten Enzyme schädigen, gegebenenfalls sind diese Gruppen vor oder nach der Synthese zu entfernen, zu schützen, auszuwaschen oder zu inaktivieren.

Sollte dies nicht möglich sein, kann gegebenenfalls durch eine größere verwendete Enzymmenge dieses Problem überwunden werden.

Um eine Anknüpfung des Linkers an die feste Phase zu ermöglichen, wird man einen Träger, der geeignet funktionalisiert ist oder in einer dem Fachmann bekannten Weise funktionalisiert werden kann, auswählen.

Bevorzugt geeignete Träger sind beispielsweise Chlorbenzylharz (Merrifieldharz), Rink-Harz (Novabiochem), Sieber-Harz (Novabiochem), Wang-Harz (Bachem), Tentagel-Harze (Rapp-Polymere), Pega-Harz (Polymer Laboratories) oder Polyacrylamide. 9-Fmocamino-xanthen-3-yloxy-Merrifieldharz, Phenylalaninol-2-chlortritylharz, Prolinol- 2-chlortritylharz, 5-Nitroanthranilsäure-2-chlortritylharz oder Hydrazin-2-chlortritylharz.

Besonders bevorzugt geeignete Träger sind Träger mit einer Aminogruppe zur Anknüpfung des Linkers wie beispielsweise Polyacrylamide, Pegaharze, Tentagel® S-NH₂, Aminomethyl-Polystyrol, 4-Methylbenzhydrylaminharz (= MBHA); Novasyn® TG Aminoharz, 4-(2'4'-Dimethoxyphenyl-Fmoc-aminomethyl)phenoxyacetamidonorleucylaminomethylharz, 4-(2'4'-Dimethoxyphenyl-Fmoc-aminomethyl)-phenoxyacetamido-norleucyl-MBHA-Harz, 4-(2'4'-Dimethoxyphenyl-Fmoc-aminomethyl)phenoxyharz, 9-Fmoc-amino-xanthen-3-yloxy-Merrifieldharz, Phenylalaninol-2-chlortritylharz, Prolinol-2-chlortritylharz, 5-Nitroanthranilsäure-2-chlortritylharz oder Hydrazin-2-chlortritylharz.

Unter dem Spacer (S) in den Verbindungen der allgemeinen Formeln I und II ist ein Spacer mit einer Länge, die 1 bis 30 Methylengruppen entspricht, zu verstehen. Der Spacer kann einen beliebigen Aufbau haben. Vorteilhafterweise wird mit der Länge des Spacers die Distanz zwischen der zentralen Linkerstruktur und fester Phase so eingestellt, daß die verwendeten Enzyme den Linker optimal spalten können.

Ist die reaktive Gruppe, über die der Spacer an die feste Phase gebunden wird, schon in einer räumlichen Distanz zur festen Phase wie beispielsweise bei Nova Syn® TG Bromo-Resin (= schwach vernetztes Polystyrolharz mit Polyethylenglycolschwänzen von 3000 - 4000 MW terminal funktionalisiert mit Brom) über Polyethylenglycolketten oder bei Rink Amide MBHA-Resin [= 4(2'4'-Dimethoxyphenyl-Fmoc-aminomethyl)phenoxyacetamido-norleucyl-4-Methylbenzhydrylamin-Harz], so kann der Spacer vorteilhafterweise entsprechend kürzer gewählt werden. Ist diese reaktive Gruppe direkt am Träger, sollte der Spacer vorteilhafterweise entsprechend länger gewählt werden. Der Spacer kann eine beliebige Struktur haben, die von untergeordneter Bedeutung ist. Die in der Struktur sowie die in den ggf. vorhandenen Substituenten enthaltenen Gruppen sollten jedoch zweckmäßigerweise nicht mit der durchgeführten Synthesechemie interferieren.

Das Grundgerüst bzw. Rückgrat des Spacers kann beispielsweise aus einer ggf. substituierten Polymethylenkette bestehen, welche anstelle einer oder mehrerer Methylengruppen Reste wie Heteroatome wie N, O, S, P, Sn oder Si oder ggf. substituierte aliphatische oder aromatische Ringe oder Ringsysteme, die ggf. weitere Heteroatome wie N, S oder 0 enthalten können, enthält.

Auch Kombinationen der genannten Reste können im Spacergrundgerüst enthalten sein.

Der Spacer wird über mindestens eine Bindung ausgewählt aus der Gruppe Ester-, Ether-, Amid-, Amin-, Sulfid- oder Phosphatbindung an die feste Phase gebunden.

Als Rest R in den Verbindungen der Formel I und II seien Wasserstoff oder ein unter den Reaktionsbedingungen inerter Rest oder zwei benachbarte inerte Reste R, die zusammen einen aromatischen, heteroaromatischen oder aliphatischen Ring bilden können, genannt. Unter inerte Reste sind beliebige aliphatische, aromatische oder heteroaromatische Reste oder Mischungen dieser Reste zu verstehen.

Als aliphatische Reste seien beispielsweise gegebenenfalls substituiertes C₁-C₈-Alkyl, C₂-C₈-Alkenyl, C₃-C₆-Alkinyl oder Cycloalkyl genannt.

Als Alkylreste seien verzweigte oder unverzweigte C₁-C₈-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl oder n-Octyl genannt.

Als Alkenylreste seien verzweigte oder unverzweigte C₃-C₈-Alkenylketten wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-5 butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl oder 7-Octenyl genannt.

Unter Alkinyl sind C₃-C₆-Alkinyl-Reste wie Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methylbut-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl zu verstehen.

Als Cycloalkylreste seien verzweigte oder unverzweigte C₃-C₁₀-Cycloalkylketten mit 3 bis 7 Kohlenstoffatomen im Ring, der Heteroatome wie S, N oder O enthalten kann, oder Ringsystem wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl, Cyclooctyl, Cyclononyl oder Cyclodecyl genannt.

Als Substituenten kommen ein oder mehrere inerte Substituenten wie Halogen, Alkyl, Aryl, Alkoxy, Benzyloxy oder Benzyl in Frage.

Unter aromatischen Resten sind einfache oder kondensierte Ringsysteme zu verstehen. Bevorzugte Reste sind Phenyl oder Naphthyl.

Heteroaromatische Reste sind vorteilhafterweise einfache oder kondensierte aromatische Ringsysteme mit einem oder mehreren heteroaromatischen 3- bis 7gliedrigen Ringen. Als Heteroatome können ein oder mehrere Stickstoff-, Schwefel- und/oder Sauerstoffatome im Ring oder Ringsystem enthalten sein.

Als Substituenten der aromatischen oder heteroaromatischen Reste kommen ein oder mehrere Substituenten wie Halogen, Alkyl, Aryl, Alkoxy, Benzyloxy oder Benzyl in Frage.

Zwei benachbarte Reste R können zusammen einen aromatischen, heteroaromatischen oder aliphatischen ggf. substituierten 4- bis 8gliedrigen Ring bilden.

Die Variable n in den Verbindungen der Formeln I und II hat die Bedeutung eins oder zwei.

Für R¹ in den Verbindungen der Formeln I und II seien als Reste substituiertes oder unsubstituiertes C₁-C₂₀-Alkyl, C₃-C₂₀-Alkenyl, C₃-C₆-Alkinyl, C₁-C₂₀-Alkylcarbonyl-, C₁-C₂₀-Alkylphosphoryl-, C₃-C₂₀-Alkenylcarbonyl-, C₃-C₆-Alkinylcarbonyl-, C₃-C₂₀-Alkenylphosphoryl-, C₃-C₆-Alkinylphosphoryl-, C₃-C₂₀-Cycloalkyl-, C₃-C₂₀-Cycloalkylcarbonyl-, C₃-C₃₀-Cycloalkylphosphoryl-, Aryl-, Arylcarbonyl-, Arylphosphoryl, Hetaryl-, Hetarylcarbonyl-, Hetarylphosphoryl-, Glycosyl-, substituierte oder unsubstituierte Aminosäuren oder Peptide genannt, wobei
- Alkyl verzweigte oder unverzweigte C₁-C₂₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosenyl;
- Alkenyl verzweigte oder unverzweigte C₃-C₂₀-Alkenyl-wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl, Dekenyl, Undekenyl, Dodekenyl, Tridekenyl, Tetradekenyl, Pentadekenyl, Hexadekenyl, Heptadekenyl, Octadekenyl, Nonadekenyl oder Eicosenyl;
- Alkinyl, verzweigte oder unverzweigte C₂-C₆-Alkinyl-Reste wie Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- Alkylcarbonyl verzweigte oder unverzweigte C₁-C₂₀-Alkylcarbonylketten mit Alkylgruppen, wie vorstehend für R¹ definiert, welche über eine Carbonylgruppe [-(C=O)-] an das Gerüst gebunden sind;
- Alkylphosphoryl verzweigte oder unverzweigte C₁-C₂₀-Alkylphosphoryl mit Alkylgruppen, wie vorstehend für R¹ definiert, welche über eine Phosphorylgruppe [-O-P(O)(OH)-] an das Gerüst gebunden sind;
- Alkenylcarbonyl verzweigte oder unverzweigte C₃-C₂₀-Alkenylcarbonylketten mit Alkenylgruppen, wie vorstehend für R¹ definiert, welche über eine Carbonylgruppe [-(C=O)-] an das Gerüst gebunden sind;
- Alkenylphosphoryl verzweigte oder unverzweigte C₃-C₂₀-Alkenylphosphorylketten mit Alkenylgruppen, wie vorstehend für R¹ definiert, welche über eine Phosphorylgruppe [-O-P(O)(OH)-) an das Gerüst gebunden sind;
- Alkinylcarbonyl verzweigte oder unverzweigte C₃-C₆-Alkinylcarbonylketten mit Alkinylgruppen, wie vorstehend für R¹ definiert, welche über eine Carbonylgruppe [-(C=O)-] an das Gerüst gebunden sind;
- Alkinylphosphoryl verzweigte oder unverzweigte C₃-C₆-Alkinylphosphorylketten mit Alkinylgruppen, wie vorstehend für R¹ definiert, welche über eine Phosphorylgruppe [-O-P(O)OH-] an das Gerüst gebunden sind;
- Cycloalkyl verzweigte oder unverzweigte C₃-C₂₀-Cycloalkylketten mit 3 bis 7 Kohlenstoffatomen im Ring, der Heteroatome wie S, N oder O enthalten kann, oder Ringsysteme wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl, Cyclooctyl, Cyclononyl oder Cyclodecyl;
- Cycloalkylcarbonyl verzweigte oder unverzweigte C₃-C₂₀-Cycloalkylcarbonylketten mit 3 bis 7 Kohlenstoffatomen im Ring, der Heteroatome wie S, N oder O enthalten kann, oder Ringsysteme wie Cyclopropylcarbonyl, Cyclobutylcarbonyl, Cyclopentylcarbonyl, Cyclohexylcarbonyl, Cycloheptylcarbonyl, 1-Methylcyclopropylcarbonyl, 1-Ethylcyclopropylcarbonyl, 1-Propylcyclopropylcarbonyl, 1-Butylcyclopropylcarbonyl, 1-Pentylcyclopropylcarbonyl, 1-Methyl-1-Butylcyclopropylcarbonyl, 1,2-Dimethylcyclopropylcarbonyl, 1-Methyl-2-Ethylcyclopropylcarbonyl, Cyclooctylcarbonyl, Cyclononylcarbonyl oder Cyclodecylcarbonyl;
- Cycloalkylphosphoryl verzweigte oder unverzweigte C₃-C₂₀-Cycloalkylphosphorylketten mit 3 bis 7 Kohlenstoffatomen im Ring, der Heteroatome wie S, N oder O enthalten kann, oder Ringsysteme wie Cyclopropylphosphoryl, Cyclobutylphosphoryl, Cyclopentylphosphoryl, Cyclohexylphosphoryl, Cycloheptylphosphoryl, 1-Methylcyclopropylphosphoryl, 1-Ethylcyclopropylphosphoryl, 1-Propylcyclopropylphosphoryl, 1-Butylcyclopropylphosphoryl, 1-Pentylcyclopropylphosphoryl, 1-Methyl-1-Butylcyclopropylphosphoryl, 1,2-Dimethylcyclopropylphosphoryl, 1-Methyl-2-Ethylcyclopropylphosphoryl, Cyclooctylphosphoryl, Cyclononylphosphoryl oder Cyclodecylphosphoryl;
- Aryl wie Phenyl oder Naphthyl;
- Arylcarbonyl wie Phenylcarbonyl oder Naphthylcarbonyl;
- Arylphosphoryl wie Phenylphosphoryl oder Naphthylphosphoryl;
- Hetaryl, Hetarylcarbonyl oder Hetarylphosphoryl, die in
- ihrem Hetarylteil aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoffoder ein Schwefelatom enthalten können;
- Glycosyl, Mono-, Di- oder Oligosaccharide wie Glucose, Galactose, Mannose, Fructose, Fucose, N-Acetyl-D-Glucosamin, Maltose, Lactose, Chitobiose, Cellobiose oder Oligosaccharide in allen ihren stereoisomeren Formen (α- oder β-Konfiguration) und allen ihren möglichen Bindungstypen [α-(1,3)-, α-(1,4)-, α-(1,6)-, β-(1,2)-, β-(1,3)-, β-(1,4)-, β-(1,6)] als Homo- oder Heteromere, wobei der zum Gerüst bindende Zucker durch eine Endo- oder Exoglycosidase oder deren Gemische erkannt und die glykosidische Bindung spaltbar sein muß, bedeutet.

Unter substituierten oder unsubstituierten Aminosäuren oder Peptiden sind natürliche oder unnatürliche Aminosäuren oder Peptide, die diese enthalten, zu verstehen. Die an das Gerüst angeknüpften Aminosäuren und Peptide sind so zu wählen, daß sie durch eine Exo- oder Endopeptidase oder eine Exo- oder Endoprotease oder Gemische aus diesen abspaltbar sind.

Alle genannte Reste R¹ können ggf. weitere Substituenten tragen, soweit dadurch die Erkennungsstelle für das Enzym nicht blockiert wird.

R² bedeutet in den Verbindungen der Formeln I und III eine nucleofuge Gruppe, welche die Anknüpfung weiterer beliebiger Reste über eine nucleophile Gruppe an den erfindungsgemäßen Linker erlaubt und dadurch eine anschließende kombinatorischen Chemie an der Festphase ermöglicht.

Als nucleofuge Gruppen seien Fluchtgruppen wie Halogen wie Br, Cl oder F oder Gruppen wie 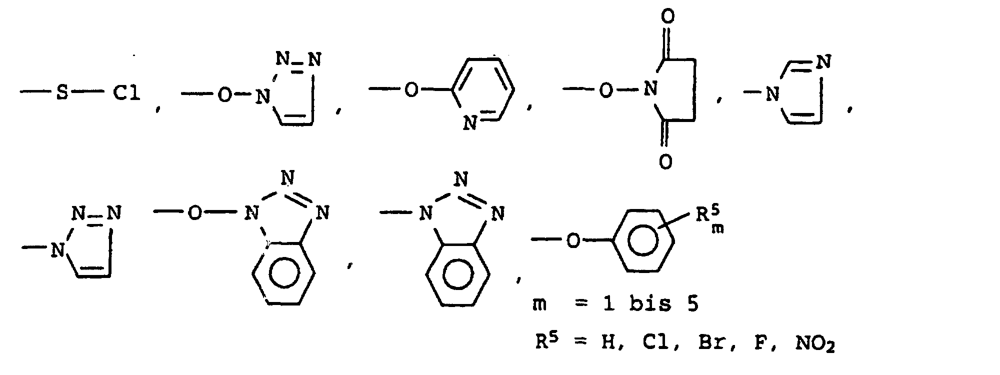 genannt.

Y in den Verbindungen der Formel III hat die für R² genannte Bedeutung und kann gleich oder verschieden von R² sein.

X bedeutet in den Verbindungen der Formel II eine der folgenden Gruppen -(C=O)-O- -O-, -NR³-, -S-, -OPO(OR⁴)-O-, wobei R³ und R⁴ unabhängig voneinander Wasserstoff oder C₁-C₈-Alkyl wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl oder n-Octyl bedeuten.

Über die Gruppe XH wird der Linker an die feste Phase (P) gebunden. Alle Gruppen, die diese Bindung ermöglichen, sind für die Synthese geeignet.

Der erfindungsgemäß festphasengebundene Linker wird vorteilhafterweise in einer Reaktionssequenz aufgebaut, die im folgenden beispielhaft für Tentagel® S-NH₂ als Träger und zwei verschiedenen Linkerkonstruktionen dargestellt wird (Schema I und II).

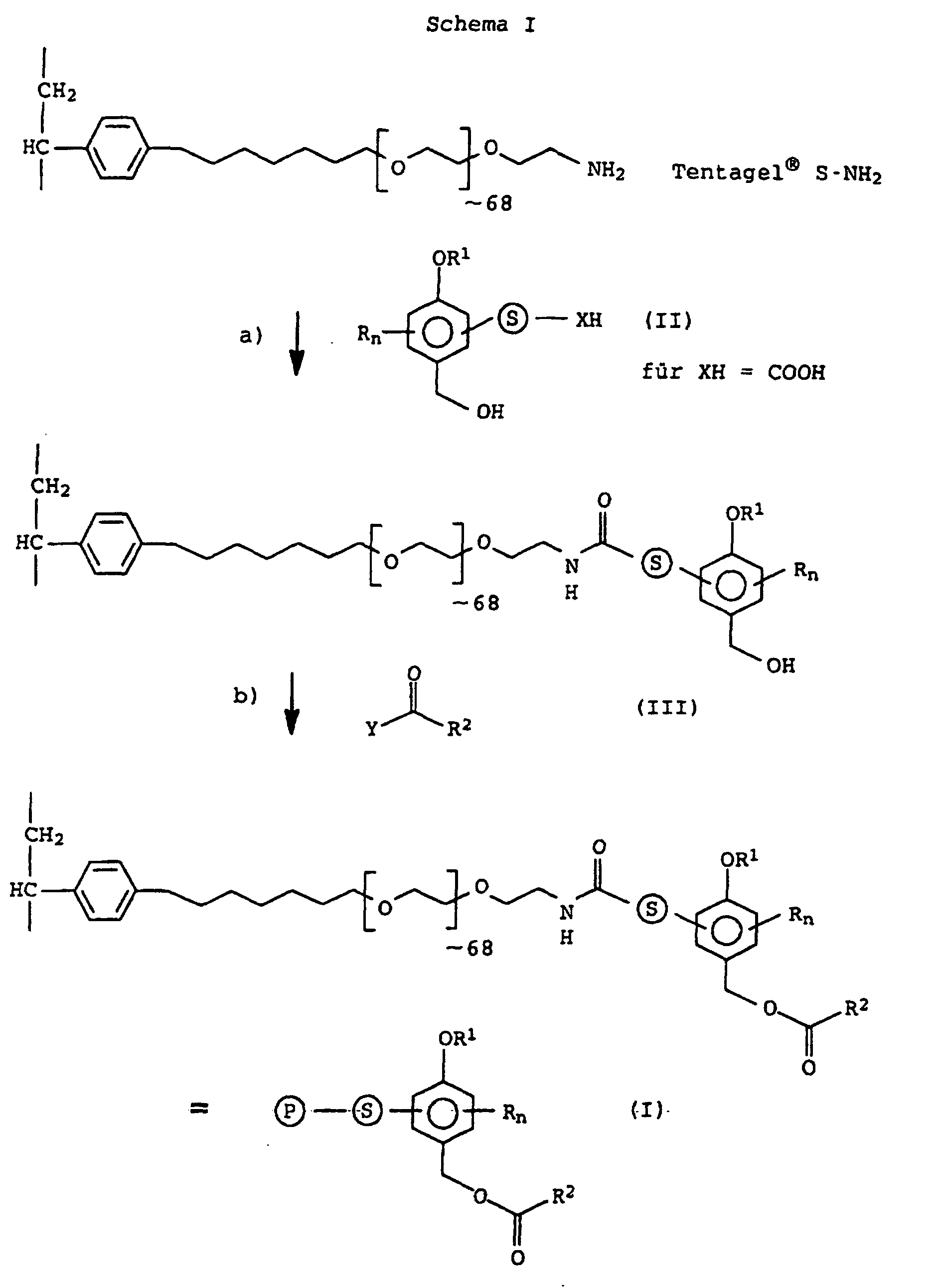

Die Anknüpfungsreaktionen a) zwischen dem Träger und Verbindungen der Formel II werden beispielsweise, wenn XH ein Carbonsäurerest ist, in Lösungsmittel mit Hilfe von beispielsweise Diisopropylcarbodiimid (= DIC) durchgeführt. Weitere zur Knüpfung dieser Amidbindung geeignete Kupplungsreagenzien sind beispielsweise TBTU, HBTS, BOP oder PYBOP (Lit.: Int. J. Peptide Prot. Rev. 35, 1990: 161-214). Als Lösungsmittel eignen sich aprotische, unpolare oder polare Lösungsmittel, beispielsweise Dimethylformamid (DMF), Methylenchlorid (CH₂Cl₂), Dimethylsulfoxid (DMSO) oder Tetrahydrofuran (THF). Es können einzelne Lösungsmittel oder Gemische verwendet werden. Die Hydroxymethylengruppe der Formel II ist zur Anknüpfung an den Linker ggf. zu schützen.

Mit der Reaktion b) wird die nucleofuge Gruppe, welche die Anknüpfung weiterer Moleküle, die dann später kombinatorisch derivatisiert werden, ermöglicht, in den Linker eingebracht.

Reaktion b) wird vorteilhafterweise mit Phosgen oder Phosgenäquivalenten in einem aprotischen polaren oder unpolaren Lösungsmittel wie CH₂Cl₂, DMF, DMSO, THF, Toluol, Acetonitril oder deren Gemische durchgeführt.

Y und R² sowie die weiteren genannten Reste in Schema I in den Formeln I bis III haben die oben genannte Bedeutung.

Beide Reaktionen werden in einem Temperaturbereich zwischen -20°C bis +120°C bevorzugt zwischen 0°C bis +60°C durchgeführt, ggf. kann Reaktion b) in Gegenwart katalytischer Mengen DMAP (= 4-Dimethylaminopyridin) durchgeführt werden.

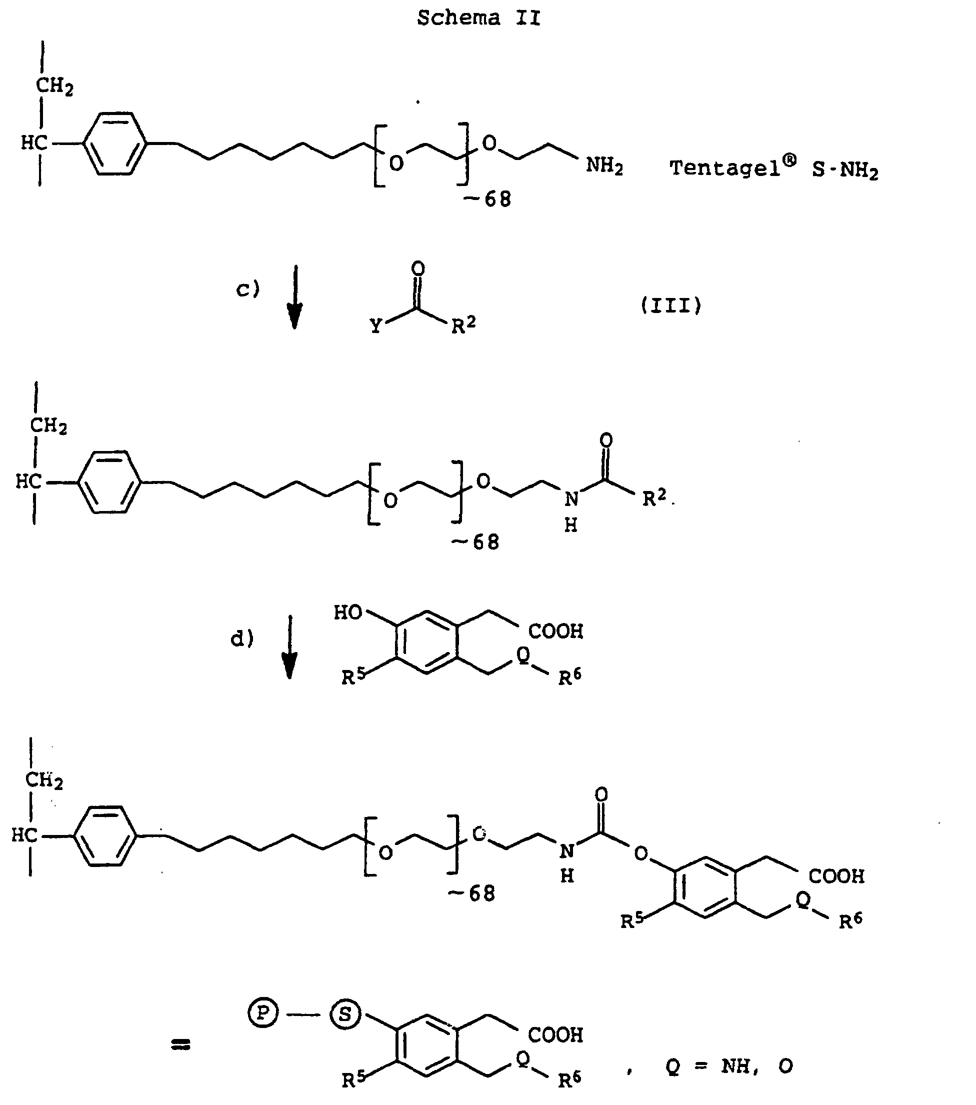

Zur Anknüpfung des Linkers an die feste Phase bzw. den Träger (siehe Schema II) wird diese zunächst funktionalisiert. Dazu wird der Träger mit einer Verbindung der allgemeinen Formel III (siehe Reaktion c) umgesetzt, worin die Reste R² und y die oben genannte Bedeutung haben. Für die Funktionalisierung des Trägers sind alle Phosgenäquivalente geeignet. Sie führen zur Einführung einer nucleofugen Gruppe, über die der Linker an den Träger gebunden wird.

Reaktion c) wird in aprotischen polaren oder unpolaren Lösungsmitteln wie CH₂Cl₂, DMF, DMSO, THF, Toluol, Acetonitril oder deren Gemische durchgeführt.

Die Reaktion wird in einem Temperaturbereich zwischen-20°C bis +120°C, bevorzugt zwischen 0°C bis +60°C, durchgeführt, ggf. kann die Reaktion in Gegenwart katalytischer Mengen DMAP durchgeführt werden. Über die nucleofuge Gruppe R² wird schließlich der Linker an den Träger gebunden (Reaktion d).

Reaktion d) wird in aprotischen polaren oder unpolaren Lösungsmitteln wie CH₂Cl₂, DMF, DMSO, THF, Toluol, Acetonitril oder deren Gemische durchgeführt.

Die Reaktion wird in Gegenwart einer tert.-Aminbase wie Triethylamin- oder Diisopropylethylamin und katalytischen Mengen DMAP durchgeführt. Reaktion d) wird in einem Temperaturbereich zwischen 0°C bis +120°C, bevorzugt zwischen 20°C bis 80°C durchgeführt.

Die Reste R⁵, R⁶ und Q haben folgende Bedeutung:
- R⁵: Wasserstoff, OH, NO₂, ggf. sub. C₁-C₈-Alkyl, C₁-C₈-Alkoxy, C₃-C₁₀-Cycloalkyl, C₃-C₁₀-Cycloalkyloxy,
- R⁶: substituiertes oder unsubstituiertes C₁-C₂₀-Alkyl, C₃-C₂₀-Alkenyl, C₃-C₆-Alkinyl, C₁-C₂₀-Alkylcarbonyl, C₁-C₂₀-Alkylphosphoryl, C₃-C₂₀-Alkenylcarbonyl-, C₃-C₆-Alkinylcarbonyl-, C₃-C₂₀-Alkenylphosphoryl-, C₃-C₆-Alkinylphosphoryl-, C₃-C₂₀-Cycloalkyl-, C₃-C₂₀-Cycloalkylcarbonyl-, C₃-C₂₀-Cycloalkylphosphoryl-, Aryl-, Arylcarbonyl-, Arylphosphoryl-, Hetaryl-, Hetarylcarbonyl-, Hetarylphosphoryl-, Glycosyl-, substituierte oder unsubstituierte Aminosäuren oder Peptide
- Q: NH oder O.

Der Rest R⁶ ist so zu wählen, daß durch Enzyme erkennbare und spaltbare Erkennungsstellen entstehen. Bindungen, die durch geeignete Wahl des Restes R⁶ entstehen und das vorher genannte Kriterium erfüllen, sind beispielsweise Ester-, Ether-, Säureamid-, Phosphorsäureester- oder Glycosidbindungen. Enzyme, die diese Bindungen spalten, sind z.B. hydrolytische Enzyme wie Lipasen, Esterasen, Amidasen, Proteasen, Peptidasen, Phosphatasen, Phospholiphasen, Peroxidasen oder Glycosidasen.

Vorteil des erfindungsgemäßen Linkers sowie des erfindungsgemäßen Verfahrens ist die einfache und vollständige Abspaltung des Linkers vom Produkt.

Der erfindungsgemäße Linker ermöglicht eine breite Palette an weiter anschließender Synthesechemie, z.B. Aufbau einer sogenannten Substanzbibliothek in der kombinatorischen Chemie, die ggf. automatisiert durchgeführt werden kann. Der erfindungsgemäße Linker läßt sich vorteilhaft für Festphasensynthesen verwenden.

Die folgenden Beispiele dienen der weiteren Veranschaulichung der Erfindung, ohne sie in irgendeiner Weise einzuschränken.

### Beispiel 1

Herstellung von 2-Acetoxy-5-methyl-benzoesäure 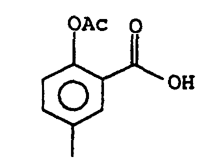 1 g 5-Methylsalicylsäure (6,58 mmol) und 1,83 ml Triethylamin (2 Äquivalente) wurden in 80 ml Essigsäureethylester gelöst. Bei 0°C wurden 0,94 ml Acetylchlorid (2 Äquivalente) zugetropft und anschließend 1 h bei 23°C gerührt. Die ausgefallenen Salze werden abfiltriert. Zu dem Filtrat wurden 100 ml 1M HCl gegeben und über Nacht gerührt. Es bildete sich ein homogene Lösung, die mit gesättigter NaHCO₃-Lösung auf etwa pH 4 eingestellt wurde. Dann wurde mit Chloroform extrahiert, die organische Phase mit wenig Wasser gewaschen und über MgSO₄ getrocknet. Das Lösungsmittel wurde weitgehend entfernt und das Rohprodukt aus Hexan/Essigsäureethylester umkristallisiert.
Ausbeute: 1,21 g (95 %)
¹H-NMR (CDCl₃): 7,89 (d,J=2 Hz); 7,39 (dd,J=8 Hz, J'=2 HZ); 6,99 (d,J=8 Hz); 2,40 (s,3 H, -CH₃); 2,31 (s,3 H, -OAc).

### Beispiel 2

Herstellung von 2-Acetoxy-5-bromomethyl-benzoesäure

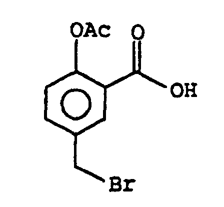

In 20 ml absolutem Tetrachlormethan wurden 1 g 2-Acetoxy-5-methyl-benzoesäure (5,16 mmol), 1,16 g N-Bromsuccinimid (1,25 Äquivalente) und 33 mg Azodiisobutyronitril unter Rühren in Argon-Atmosphäre vorsichtig erhitzt und 2,5 h unter Rückfluß und Bestrahlung mit einer Tageslichtlampe gekocht. Anschließend ließ man im Eisbad abkühlen, filtrierte ab und wusch den Filterkuchen mit n-Pentan. Der Kristallbrei wurde in CHCl₃ aufgenommen und mit kaltem Wasser gewaschen. Die organische Phase wurde über MgSO₄ getrocknet und eingeengt. Das Rohprodukt wurde direkt weiter umgesetzt.
Ausbeute: 1 g, 58 % Titelverbindung, neben etwa 10 % der 2-Acetoxy-5-dibromomethyl-benzoesäure (abgeschätzt anhand des ¹H-NMR-Spektrums).
¹H-NMR (CDCl₃): 8,11 (d,J=2 Hz); 7,62 (dd,J=8 Hz, J'=2 Hz); 7,11 (d,J=8 Hz); 4,48 (s,2 H, -CH₂-); 2,33 (s,3 H, -OAc).

### Beispiel 3

Herstellung von 2-Acetoxy-5-hydroxymethyl-benzoesäure 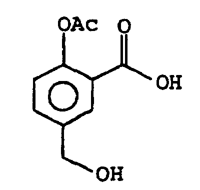 1 g des Rohprodukts (2,99 mmol 4) aus Beispiel 2 wurden mit 25,5 ml Dioxan und 30 ml 0,1 N AgNO₃-Lösung versetzt und über Nacht bei Raumtemperatur gerührt. Dann wurde mehrfach mit Essigsäureethylester extrahiert, die vereinigten organischen Phasen über MgSO₄ getrocknet und bis zur Trockne eingeengt. Säulenchromatographie mit CHCl₃/Methanol (5:1) ergab eine Ausbeute von 389 mg (62 %).
¹H-NMR (CDCl₃): 8,07 (d.J=2 Hz); 7,61 (dd,J=8 Hz, J'=2 Hz); 7,12 (d,J=8 Hz); 4,73 (s,2 H, -CH₂); 2,32 (s,3 H, -OAc).

### Beispiel 4

Anbinden des Ankerbausteins 2-Acetoxy-5-hydroxymethyl-benzoesäure an TentaGel® S-NH₂

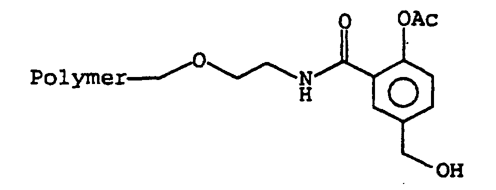

Zu 500 mg TentaGel® S-NH₂ (0,29 mmol NH₂-Gruppen/g) in 6 ml absolutem CH₂Cl₂ wurden 36,5 mg 2-Acetoxy-5-hydroxymethyl-benzoesäure (1,2 Äquivalente) und 21,5 µl Diisopropylcarbodiimid (1,44 Äquivalente) gegeben. Das Reaktionsgemisch wurde bei Raumtemperatur gerührt und das Harz auf eine Fritte abgesaugt. Sukzessive wurde dann jeweils dreimal mit absolutem DMF, Methanol und CH₂Cl₂ auf der Fritte gewaschen, wobei mit durchgeleitetem Stickstoff durchmischt wurde. Das Harz wurde im Ölpumpenvakuum getrocknet und das Anbindungsprotokoll einmal wiederholt, bis der Kaiser-Test auf freie NH₂-Gruppen negativ ausfällt (E. Kaiser et al. Anal. Biochem. 1979, 34, 595).
FT-IR: 1766 cm⁻¹ (-OAc), 1666 cm⁻¹ (-CONH-), 1540 cm⁻¹ (-NH-), 3100-3500 cm⁻¹ (-OH).

### Beispiel 5

Umsetzung zum Chlorameisensäureester 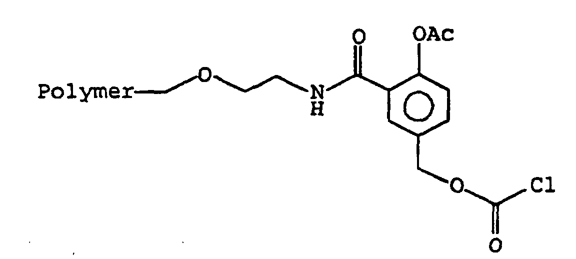 210 mg des aus Beispiel 4 gewonnenen Produktes wurden in 3,5 ml absolutem THF unter Argon vorgelegt und 400 µl (etwa 12 Äquivalente) einer Lösung von Phosgen in Toluol (1,93 M) bei 23°C zugetropft. Die Suspension wurde 2 h gerührt und nach tropfenweiser Zugabe von weiteren 200 µl der Phosgenlösung 2 h weitergerührt. Anschließend wurde nacheinander jeweils zweimal mit absolutem THF, Ethylether, THF und nochmals Ethylether gewaschen und das Harz im Vakuum getrocknet.
FT-IR: 1770,5 cm⁻¹ (-OAc und -COCl), 1666 cm⁻¹ (-CONH-), 1540 cm⁻¹ (-NH-).

### Beispiel 6

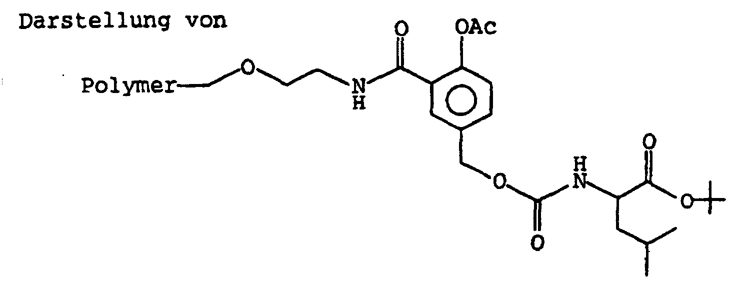

durch Kopplung mit Leucin-tert.-butylester Hydrochlorid.

Eine im Eiswasserbad gekühlte Lösung von 29 mg Leucin-tert.butylester Hydrochlorid (4 Äquivalente) und 20 µl Triethylamin (etwa 5 Äquivalente) in 4 ml absolutem CH₂Cl₂ wurde bei 0°C unter Argon langsam zu einer Suspension von 110 mg der gemäß Beispiel 5 erhaltenen Verbindung in 2 ml CH₂Cl₂ gegeben. Die Suspension wurde 0,5 h bei 0°C und 3 h bei 23°C gerührt. Dann wurde nacheinander jeweils mit absolutem Methanol, CH₂Cl₂, Methanol und Ethylether gewaschen und das Harz im Vakuum getrocknet.
FT-IR: 1776 cm⁻¹ (-OAc), 1724 cm⁻¹ (-OCONH-), 1666 cm⁻¹ (-CONH-), 1540 cm⁻¹ (-NH-).

### Beispiel 7

### Basische Abspaltung des Leucin-tert.-butylesters zur Bestimmung der Belegungsdichte

6 mg des Kopplungsproduktes aus Beispiel 6 wurden in einer Lösung von 200 µl THF und 200 µl gesättigter NaHCO₃-Lösung, pH 10,5, suspendiert und das Reaktionsgemisch etwa 30 min bei 23°C geschüttelt. Danach wurde mit 200 µl CHCl₃ extrahiert und die organische Phase per GC-MS anhand einer Eichgrade quantifiziert. Die Anbindungsrate von Leucin-tert.-butylester über den Ankerbaustein an den polymeren Träger bezüglich der verfügbaren NH₂-Gruppen betrug 51 %.

### Beispiel 8

### Enzymatische Abspaltung des Leucin-tert.-butylesters

27 mg des Kopplungsproduktes aus Beispiel 6 wurden in 1066 µl Phosphat-Puffer (0,1 M Na₂HPO₄, 0,2 M KI, pH 5) und 500 µl Methanol suspendiert; 3 U Mucor miehei-Lipase, gelöst in 100 µl des gleichen Puffers, wurden zugegeben und der Ansatz bei 30°C unter Schütteln inkubiert. Durch Anwesenheit von Iodid in der Pufferlösung wurde das intermediär entstehende Chinonmethin abgefangen. Nach 6 h und 32 h wurden jeweils weitere 100 µl der Lipaselösung zugefügt. Die Inkubation wurde nach 58 h abgebrochen. Das Reaktionsgemisch wurde mit CHCl₃ extrahiert.
Die vereinigten und getrockneten organischen Phasen wurden auf 1 ml eingeengt und per GC-MS quantifiziert.
Ausbeute: 42 %

## Patentansprüche

1. Enzymatisch spaltbarer Linker, gebunden an eine feste Phase, an dem über eine funktionelle Gruppe organische Verbindungen synthetisiert werden, **dadurch gekennzeichnet, daß** der Linker eine Erkennungsstelle für ein hydrolytisches Enzym enthält und durch Reaktion mit dem Enzym so zerfällt, daß keine Molekülteile des Linkers im synthetisierten Produkt verbleiben und daß die Erkennungsstelle für das Enzym und die Stelle, an der das Syntheseprodukt über Zerfall des Linkers freigesetzt wird, verschieden sind.

2. Linker nach Anspruch 1, **dadurch gekennzeichnet, daß** der Linker unter CO₂-Abspaltung vom an der Festphase synthetisierten Produkt abgespalten wird.

3. Festphasen-gebundener Linker nach Anspruch 1 oder 2, **dadurch gekennzeichnet, daß** der Linker eine Erkennungsstelle für ein hydrolytisches Enzym ausgewählt aus der Gruppe Lipasen, Esterasen, Amidasen, Proteasen, Peptidasen, Phosphatasen, Phospholipasen, Peroxidasen oder Glycosidasen enthält.

4. Linker nach den Ansprüchen 1 bis 3 der allgemeinen Formel I 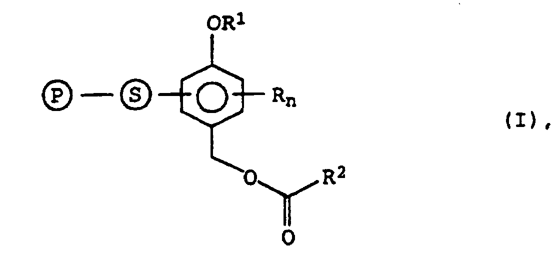 in der die Variablen und Substituenten folgende Bedeutung haben:
(P) eine feste Phase
(S) ein Spacer, mit einer Länge, die 1 bis 30 Methylengruppen entspricht
R Wasserstoff oder ein unter den Reaktionsbedingungen inerter Rest oder zwei benachbarte inerte Reste R, die zusammen einen aromatischen, heteroaromatischen oder aliphatischen Ring bilden
R¹ substituiertes oder unsubstituiertes C₁-C₂₀-Alkyl-, C₃-C₂₀-Alkenyl-, C₃-C₆-Alkinyl-, C₁-C₂₀-Alkylcarbonyl-, C₁-C₂₀-Alkylphosphoryl-, C₃-C₂₀-Alkenylcarbonyl-, C₃-C₆-Alkinylcarbonyl-, C₃-C₂₀-Alkenylphosphoryl-, C₃-C₆-Alkinylphosphoryl-, C₃-C₂₀-Cycloalkyl-, C₃-C₂₀-Cycloalkylcarbonyl-, C₃-C₂₀-Cycloalkylphosphoryl-, Aryl-, Arylcarbonyl-, Arylphosphoryl-, Hetaryl-, Hetarylcarbonyl-, Hetarylphosphoryl-, Glycosyl-, substituierte oder unsubstituierte Aminosäuren oder Peptide
R² eine nucleofuge Gruppe
n 1 oder 2

5. Linker der Formel I nach den Ansprüchen 1 bis 4, wobei die feste Phase (P) funktionalisierte Partikel ausgewählt aus der Gruppe Keramik, Glas, Latex, quervernetzte Polystyrole, quervernetzte Polyacrylamide oder andere Harze, natürliche Polymere, Gold, colloidale Metallpartikel, Silicagele, Aerogele oder Hydrogele bedeutet.

6. Verfahren zur Herstellung eines Linkers der Formel I an fester Phase, 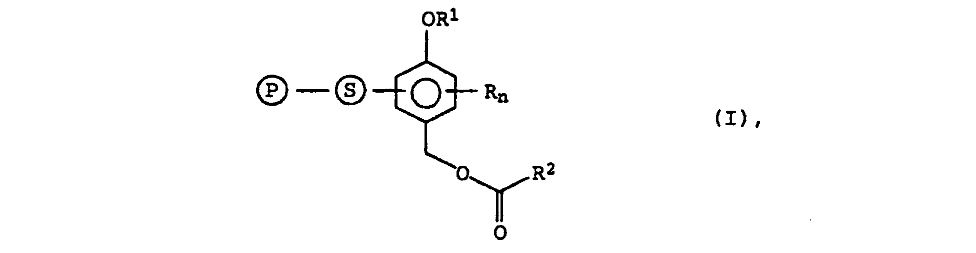 in der die Variablen und Substituenten folgende Bedeutung haben:
(P) eine feste Phase
(S) ein Spacer, mit einer Länge, die 1 bis 30 Methylengruppen entspricht
R Wasserstoff oder ein unter den Reaktionsbedingungen inerter Rest
oder zwei benachbarte inerte Reste R, die zusammen einen aromatischen, heteroaromatischen oder aliphatischen Ring bilden
R¹ substituiertes oder unsubstituiertes C₁-C₂₀-Alkyl-, C₃-C₂₀-Alkenyl-, C₃-C₆-Alkinyl-, C₁-C₂₀-Alkylcarbonyl-, C₁-C₂₀-Alkylphosphoryl-, C₃-C₂₀-Alkenylcarbonyl-, C₃-C₆-Alkinylcarbonyl-, C₃-C₂₀-Alkenylphosphoryl-, C₃-C₆-Alkinylphosphoryl-, C₃-C₂₀-Cycloalkyl-, C₃-C₂₀-Cycloalkylcarbonyl-, C₃-C₂₀-Cycloalkylphosphoryl-, Aryl-, Arylcarbonyl-, Arylphosphoryl-, Hetaryl-, Hetarylcarbonyl-, Hetarylphosphoryl-, Glycosyl-, substituierte oder unsubstituierte Aminosäuren oder Peptide
R² eine nucleofuge Gruppe
n 1 oder 2
**dadurch gekennzeichnet, daß** man Verbindungen der Formel II 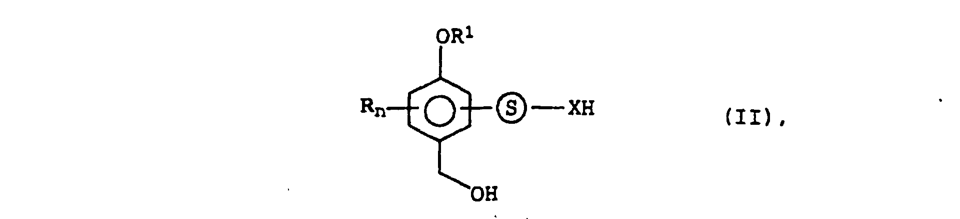 worin X für COO, O, NR³, S, OPO(OR⁴)-O steht und R³, R⁴, unabhängig voneinander Wasserstoff, C₁-C₈-Alkyl- bedeutet, über eine Ester-, Ether-, Amid-, Amin-, Sulfid- oder Phosphatbindung an die feste Phase (P) bindet und anschließend mit Verbindungen der Formel III 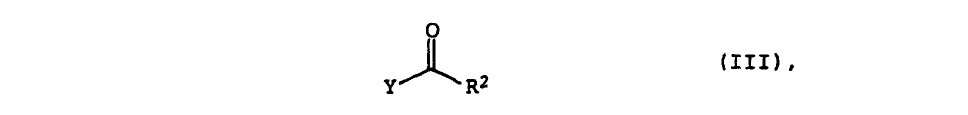 wobei Y die Bedeutung von R² hat und gleich oder verschieden ist, umsetzt.

7. Verwendung von Linkern gemäß den Ansprüchen 1 bis 5 in der Festphasensynthese.

8. Verwendung von Verbindungen gemäß Anspruch 4 der Formel I in der Festphasensynthese.

## Claims

1. An enzymatically cleavable linker which is bound to a solid phase and on which organic compounds are synthesized via a functional group, wherein the linker contains a recognition site for a hydrolytic enzyme and is fragmented by reaction with the enzyme in such a way that no parts of the linker molecule remain in the synthesized product, and wherein the recognition site for the enzyme and the site at which the synthetic product is liberated by fragmentation of the linker are different.

2. A linker as claimed in claim 1, wherein the linker is eliminated from the product synthesized on the solid phase with elimination of CO₂.

3. A linker as claimed in claim 1 or 2, which is bound to a solid phase, wherein the linker contains a recognition site for a hydrolytic enzyme selected from the group of lipases, esterases, amidases, proteases, peptidases, phosphatases, phospholipases, peroxidases or glycosidases.

4. A linker as claimed in any of claims 1 to 3 of the formula I 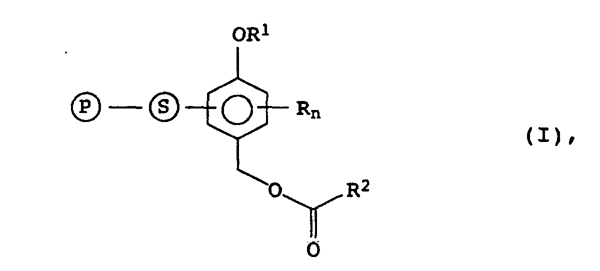 in which the variables and substituents have the following meanings:
(P) a solid phase
(S) a spacer with a length equivalent to 1 to 30 methylene groups
R hydrogen or a radical which is inert under the reaction conditions
or two adjacent inert radicals R which together form an aromatic, heteroaromatic or aliphatic ring
R¹ substituted or unsubstituted C₁-C₂₀-alkyl, C₃-C₂₀-alkenyl, C₃-C₆-alkynyl, C₁-C₂₀-alkylcarbonyl, C₁-C₂₀-alkylphosphoryl, C₃-C₂₀-alkenylcarbonyl, C₃-C₆-alkynylcarbonyl, C₃-C₂₀-alkenylphosphoryl, C₃-C₆-alkynylphosphoryl, C₃-C₂₀-cycloalkyl, C₃-C₂₀-cycloalkylcarbonyl, C₃-C₂₀-cycloalkylphosphoryl, aryl, arylcarbonyl, arylphosphoryl, hetaryl, hetarylcarbonyl, hetarylphosphoryl, glycosyl, substituted or unsubstituted amino acids or peptides
R² a nucleofugic group
n 1 or 2.

5. A linker of the formula I as claimed in any of claims 1 to 4, where the solid phase (P) means functionalized particles selected from the group of ceramics, glass, latex, crosslinked polystyrenes, crosslinked polyacrylamides or other resins, natural polymers, gold, colloidal metal particles, silica gels, aerogels or hydrogels.

6. A process for preparing a linker of the formula I on a solid phase, 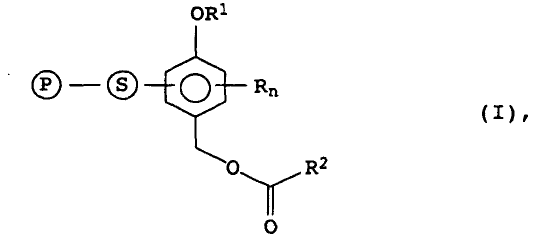 in which the variables and substituents have the following meanings:
(P) a solid phase
(S) a spacer with a length equivalent to 1 to 30 methylene groups
R hydrogen or a radical which is inert under the reaction conditions or two adjacent inert radicals R which together form an aromatic, heteroaromatic or aliphatic ring
R¹ substituted or unsubstituted C₁-C₂₀-alkyl, C₃-C₂₀-alkenyl, C₃-C₆-alkynyl, C₁-C₂₀-alkylcarbonyl, C₁-C₂₀-alkylphosphoryl, C₃-C₂₀-alkenylcarbonyl, C₃-C₆-alkynylcarbonyl, C₃-C₂₀-alkenylphosphoryl, C₃-C₆-alkynylphosphoryl, C₃-C₂₀-cycloalkyl, C₃-C₂₀-cycloalkylcarbonyl, C₃-C₂₀-cycloalkylphosphoryl, aryl, arylcarbonyl, arylphosphoryl, hetaryl, hetarylcarbonyl, hetarylphosphoryl, glycosyl, substituted or unsubstituted amino acids or peptides
R² a nucleofugic group
n 1 or 2,
which comprises linking compounds of the formula II 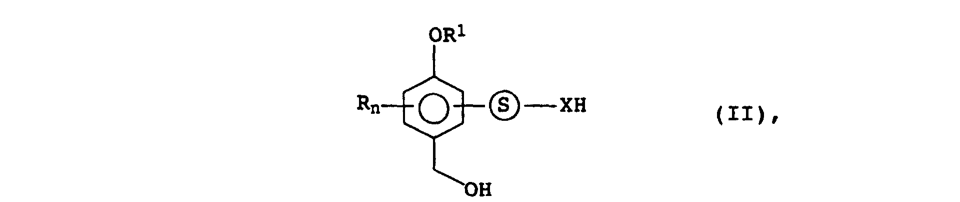 where X is COO, O, NR³, S, OPO(OR⁴)-O and R³, R⁴, are, independently of one another, hydrogen, C₁-C₈-alkyl, via an ester, ether, amide, amine, sulfide or phosphate linkage to the solid phase (P), and subsequently reacting with compounds of the formula III 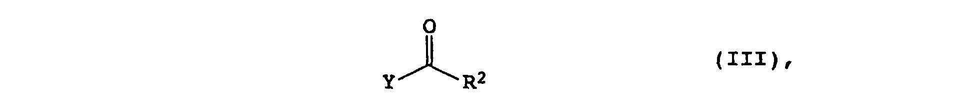 where Y has the meaning of R² and is identical or different.

7. The use of a linker as claimed in any of claims 1 to 5 in solid-phase synthesis.

8. The use of a compound as claimed in claim 4 of the formula I in solid-phase synthesis.

## Revendications

1. Chaînon relié à une phase solide mais qui peut en être scindé sous l'action d'enzymes, et sur lequel, en raison de la présence d'un groupe fonctionnel, on procède à la synthèse de composés organiques, ce chaînon **se caractérisant en ce qu'**il contient un site de reconnaissance pour une enzyme hydrolytique et se décompose par réaction avec l'enzyme sans qu'il subsiste dans le produit synthétisé aucune fraction de la molécule du chaînon, et **en ce que** le site de reconnaissance pour l'enzyme et le site où le produit de synthèse est libéré par décomposition du chaînon sont différents.

2. Chaînon selon la revendication 1, **caractérisé en ce qu'**il est séparé du produit synthétisé sur la phase solide avec libération de CO₂.

3. Chaînon fixé sur une phase solide selon les revendications 1 ou 2, **caractérisé en ce qu'**il contient un site de reconnaissance pour une enzyme hydrolytique choisie dans le groupe consistant en les lipases, les estérases, les amidases, les protéases, les peptidases, les phosphatases, les phospholipases, les peroxydases et les glycosidases.

4. Chaînon selon les revendications 1 à 3, répondant à la formule générale (I) 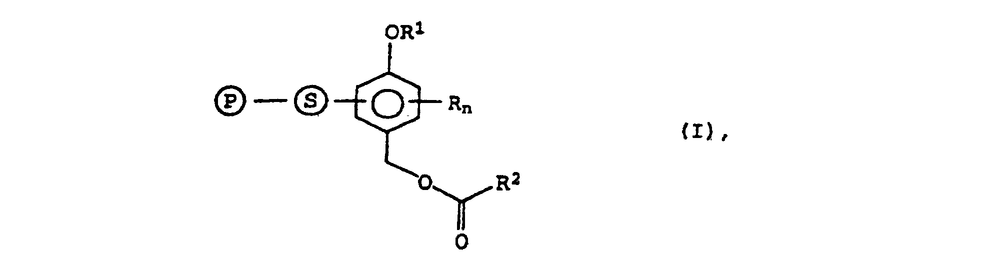 dans laquelle les symboles et indices ont les significations suivantes :
(P) une phase solide,
(S) un espaceur dont la longueur correspond à 1 à 30 groupes méthylènes,
R l'hydrogène on un groupe inerte dans les conditions de réaction, ou bien deux groupes R inertes et voisins forment un cycle aromatique, hétéro-aromatique ou aliphatique,
R¹ des aminoacides ou peptides non substitués ou portant des substituants alkyle en C1-C20, alcényle en C3-C20, alcynyle en C3-C6, (alkyle en C1-C20)carbonyle, (alkyle en C1-C20) phosphoryle, (alcényle en C3-C20)carbonyle, (alcynyle en C3-C6)carbonyle, (alcényle en C3-C20)phosphoryle, (alcynyle en C3-C6)phosphoryle, cycloalkyle en C3-C20, (cycloalkyle en C3-C20)carbonyle, (cycloalkyle en C3-C20)phosphoryle, aryle, arylcarbonyle, arylphosphoryle, hétéaryle, hétéroarylcarbonyle, hétéroarylphosphoryle, glycosyle eux-mêmes substitués ou non,
R² un groupe nucléofuge,
n 1 ou 2.

5. Chaînon de formule (I) selon les revendications 1 à 4, pour lequel la phase solide (P) consiste en particules fonctionnalisées choisies dans le groupe consistant en les matières céramiques, le verre, les latex, les polystyrènes réticulés, les polyacrylamides réticulés ou d'autres résines, des polymères naturels, l'or, des particules de métaux colloïdaux, des gels de silice, des aérogels, ou des hydrogels.

6. Procédé pour la préparation d'un chaînon de formule (I) sur phase solide, 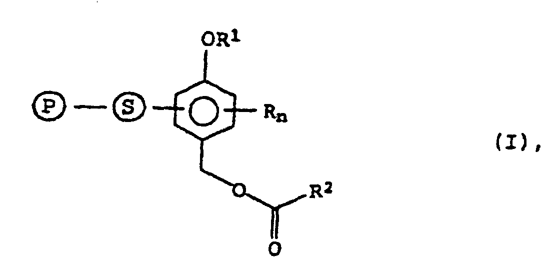 Les symboles et indices de cette formule ayant les significations suivantes :
(P) une phase solide,
(S) un espaceur dont la longueur correspond à 1 à 30 groupes méthylène,
R l'hydrogène ou un groupe inerte dans les conditions de réaction, ou bien deux groupes inertes voisins R forment ensemble un cycle aromatique hétéroaromatique ou aliphatique,
R¹ des aminoacides ou peptides non substitués ou portant des substituants alkyle en C1-C20, alcényle en C3-C20, alcynyle en C3-C6, (alkyle en C1-C20)-carbonyle, (alkyle en C1-C20)phosphoryle, (alcényle en C3-C20)carbonyle, (alcynyle en C3-C6)carbonyle, (alcényle en C3-C20)phosphoryle, (alcynyle en C3-C6)phosphoryle, cycloalkyle en C3-C20, (cycloalkyle en C3-C20)carbonyle, (cycloalkyle en C3-C20)phosphoryle, aryle, arylcarbonyle, arylphosphoryle, hétéroaryle, hétéroarylcarbonyle, hétéroarylphosphoryle, glycosyle eux-mêmes substitués ou non,
R² un groupe nucloéfuge,
n 1 ou 2,
**caractérisé en ce que** l'on fixe sur la phase solide (P), par l'intermédiaire d'une liaison ester, éther, amide, amine, sulfure ou phosphate, des composés de formule (II) 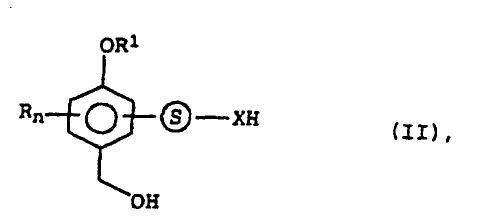 dans laquelle X représente COO, O, NR³, S, OPO(OR⁴)-O et R³ et R⁴ représentent chacun, indépendamment l'un de l'autre, l'hydrogène, un groupe alkyle en C1-C8, puis on fait réagir avec des composés de formule (III) 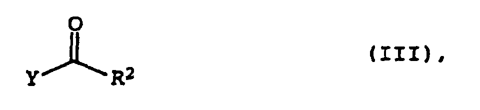 dans laquelle Y a les mêmes significations que R² mais peut être identiques à celui-ci ou différent de celui-ci.

7. Utilisation des chaînons selon les revendications 1 à 5 pour la synthèse en phase solide.

8. Utilisation des composés de formule (I) selon la revendication 4 dans des synthèses en phase solide.
